(19) Europäisches Patentamt
European Patent Office
Office européen des brevets

(11) **EP 3 868 858 A1**

(12) **EUROPÄISCHE PATENTANMELDUNG**

(43) Veröffentlichungstag:
**25.08.2021 Patentblatt 2021/34**

(51) Int Cl.:
**C12M 1/107** (2006.01)     **C12M 1/12** (2006.01)
**C12M 1/00** (2006.01)     **C12M 1/34** (2006.01)

(21) Anmeldenummer: **20158085.9**

(22) Anmeldetag: **18.02.2020**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **PRE Power Recycling Energyservice GmbH**
**17033 Neubrandenburg**
**Mecklenburg-Vorpommern (DE)**

(72) Erfinder:
• **ROSSOW, Norbert**
**17217 Penzlin (DE)**
• **AZBAR, Nuri**
**Izmir (TR)**

(74) Vertreter: **Wehlan, Helmut**
**Patentanwälte Wehlan & Wehlan**
**Möllendorffstrasse 49**
**10367 Berlin (DE)**

(54) **VERFAHREN UND VORRICHTUNG ZUR NUTZUNG VON REST-CO2 FÜR DIE WEITERE CH4-PRODUKTION**

(57) Die Erfindung betrifft ein neues Verfahren und eine entsprechende Vorrichtung für die Umwandlung von Kohlendioxid und Wasserstoff zu Methan. Mögliche Anwendungsgebiete sind die Steigerung des Methan-Anteils in Biogasanlagen auf über 85% Methan. Die Vorrichtung umfasst Reaktoren, die anaerobe methanbildende Bakterien aus Schlamm Granulat und darüber Katalysatoren/mikrobielle Besiedlungsfläche aus organischen Kunststoffen, Mineralien oder metallischen Stoffen mit spezifisch sehr großer Oberfläche aufweisen, dadurch gekennzeichnet, dass die Reaktorräume mehrfach in einer Mikro-Kolonne zu einem Mehrfach-Reaktor aufgeschichtet sind.

Figur 3

## Turbo-Methan

**Beschreibung**

Die Erfindung betrifft ein neues Verfahren und eine entsprechende Vorrichtung für die Umwandlung von Kohlendioxid und Wasserstoff zu Methan. Mögliche Anwendungsgebiete sind die Steigerung des Methan-Anteils in Biogasanlagen auf über 85% Methan.

Stand der Technik

Der Sabatier-Prozess oder die Sabatier-Reaktion, benannt nach dem französischen Chemiker Paul Sabatier und von diesem 1902 mit Jean Baptiste Senderens erfunden, [Sabatier, Senderens Compte Rendu Acad. Sci., Band 134, 1902, S. 689] beschreibt eine chemische Reaktion, bei der Kohlenstoffdioxid und Wasserstoff in Methan und Wasser umgewandelt werden. Die Reaktion wird durch folgende Reaktionsgleichung beschrieben:

$$CO_2 + 4\,H_2 \quad \rightarrow CH_4 + 2\,H_2O \qquad\qquad \Delta H^0 = \text{-}165\ kJ\,/\,mol \qquad\qquad Gl.\ 1$$

Die Reaktion verläuft stark exotherm.

Bei erhöhter Temperatur und erhöhtem Druck läuft die Reaktion unter Verwendung eines Nickel-Katalysators ab, effektiver ist die Verwendung von Ruthenium auf einem Aluminiumoxid-Substrat. Oft ist auch ein Sabatier-Prozess in Verbindung mit einer Wasserstoff-Elektrolyse technisch relevant, da sich so Methan und Sauerstoff erzeugen lassen

Die Reaktionsgleichung lautet dann

$$CO_2 + 4H_2 \rightarrow CH_4 + 2\,H_2O \rightarrow CH_4 + 2\,H_2 + O_2 \qquad\qquad Gl.\ 2$$

Ein neuer Ansatz ist die Umwandlung von Strom zu synthetischem Erdgas. Dabei wird mit überschüssigem Strom zunächst Wasserstoff durch Elektrolyse mit einem Wirkungsgrad von 57 bis 73 Prozent erzeugt. Mit dem Sabatier-Prozess wird anschließend Wasserstoff und Kohlenstoffdioxid zu Methan umgewandelt, wobei das Methan ($CH_4$) vor Ort gespeichert oder in Erdgasleitungen eingespeist und in großen Erdgasspeichern zwischengelagert werden kann. Beim Verbrennen in der z. Z. (2011) modernsten Gasturbine SGT5-8000H, ist der Wirkungsgrad 60,3 % und somit der Verlust in diesem Prozessabschnitt 39,7 %. [Matthias Brake: Erdgasleitungen als Speicher für Windenergie. In: Telepolis. Abgerufen am 18. April 2011].

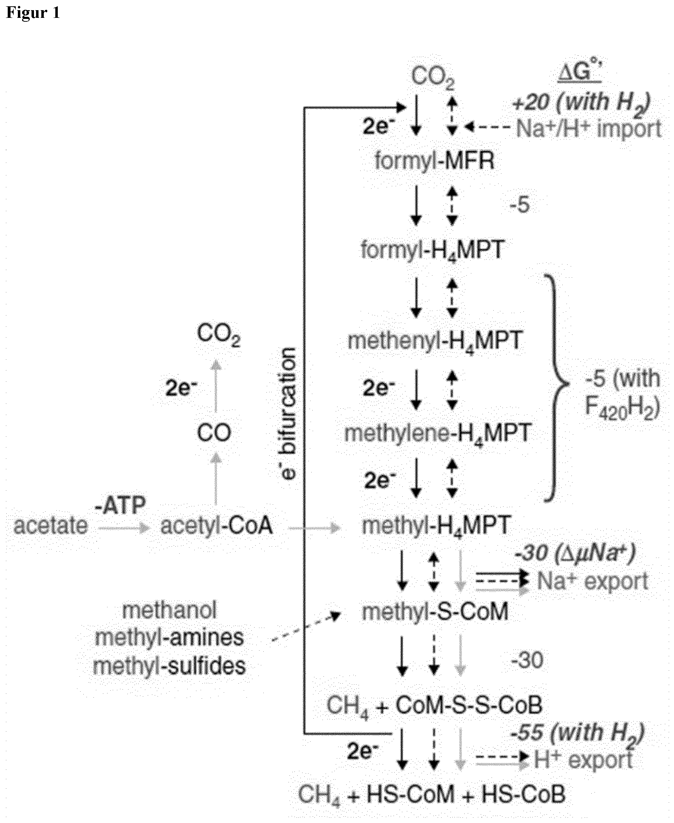

Wege der Methanogenese sind bekannt und von Costa and Leigh 2014 zusammengefasst worden (Figur 1). Die Reaktionen von hydrogenotropher (feste schwarze Linien), methylotropher (gestrichelte schwarze Linien) und acetiklastischer (graue Linien) Methanogenese sind dargestellt. (Costa K und Leigh A. Metabilic versatility in methanogens, current option in Biotechnology 2014, 29:70-75).

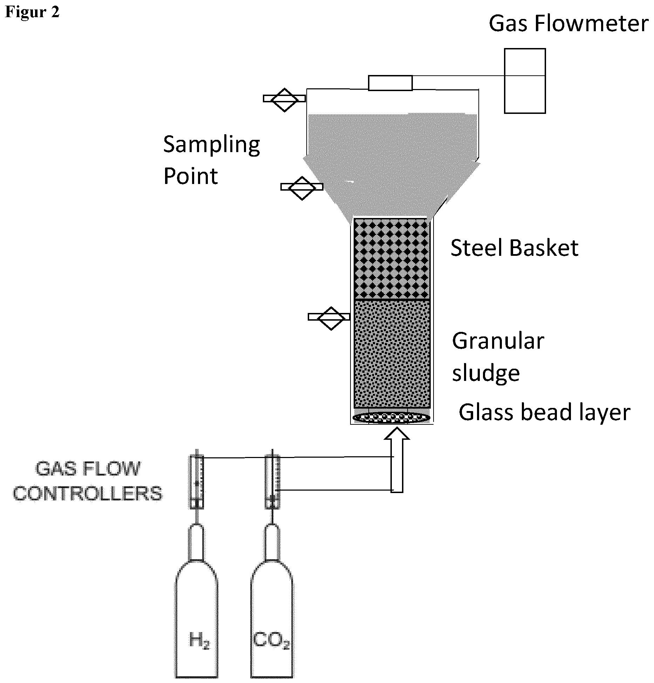

Zum Stand der Technik gehört auch die Veröffentlichung von Nuri Azbar et al., die am 28. November 2017 online auf der Web-Seite https://www.tandfonline.com/doi/full/10.1080/09593330.2017.1406537?scroll=top&needAccess=true veröffentlicht wurde. In dieser Studie wurde die anaerobe Biokonversion von $CO_2$ in Biomethan mittels einer neuartigen Bioprozesskonfiguration (HYBRID-Bioreaktor) unter mesophilen Bedingungen untersucht. Eine Abbildung des dort beschriebenen HYBRID-Bioreaktors ist in Figur 2 dargestellt.

Dieser HYBRID-Bioreaktor besteht aus gemäß Figur 2 aus einem Granulatschlamm, der die anaeroben Bakterien für die Methanisierung enthält und darüber befindet sich ein Stahlkorb mit Ringen zur Immobilisierung. Dieser Reaktor stellt aber nur einen Prototyp dar und ist nicht für eine technische Anwendung geeignet.

Nachteil der bekannten technischen Lösungen

Die bekannten technischen Lösungen arbeiten bei hohen Drücken und hohen Temperaturen mit Katalysatoren. Gemäß Gleichung 2 (siehe oben) kann es dabei zur elektrolytischen Spaltung von Wasser kommen und damit zur Erzeugung von Wasserstoff und Sauerstoff in einer Vorrichtung, wobei es zur Knallgas-Explosion kommen kann.

Die Nachteile des oben beschriebenen HYBRID-Bioreaktors sind folgende:

-    Die Ausbeute an $CH_4$ aus den Einsatzstoffen erreicht nicht mehr als 85%

-    Der realisierte Volumenstrom führt zu einem riesigen Volumenbedarf

-    Für die erforderlichen Volumina in üblichen Kolonnenreaktoren ergeben sich Probleme bzgl. der Durchdringung ausgehend vom bakteriellen Granulat-Katalysator

Aufgabe der Erfindung

Die Aufgabe der Erfindung bestand darin, die Nachteile der im Stand der Technik beschriebenen technischen Lösungen zu beseitigen.

Lösung der Aufgabe

Die Aufgabe wurden gemäß den Merkmalen der Patentansprüche gelöst. Erfindungsgemäß wird ein verbessertes Verfahren und eine entsprechende Vorrichtung zur Methanisierung von Kohlendioxid bereitgestellt. Das Verfahren läuft bei sowohl bei normalen als auch bei erhöhten Temperaturen ab.

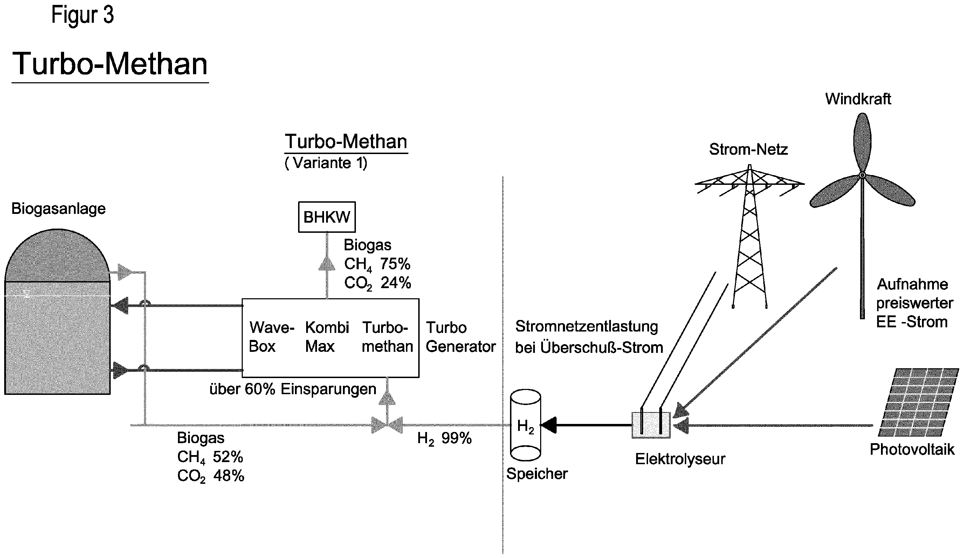

Das erfindungsgemäße Verfahren ist schematisch in Figur 3 abgebildet.

Im Unterschied zum oben beschriebenen HYBRID-Bioreaktor werden die Reaktorräume in der Kombination Granulat-Katalysator/Mikrobieller-Siedlung-Reaktionsraum mehrfach in einer Mikro-Kolonne zu einem Mehrfach-Reaktor aufgeschichtet. Die Schichtung erfolgt senkrecht übereinander in wechselbaren Einfüllschächten als Füllkörbe mit Flüssigkeits- und gasdurchlässigem Boden. Die Mikro-Kolonne besteht aus vorzugsweise rechteckigen oder zylindrischen Bauteilen.

Die Mikro-Kolonnen werden - gemäß einer bevorzugten Ausführungsform - als Einschub-System und mit Gleiteinrichtungen ausgestattetes Grundmodul hergestellt. Mit diesem Grundmodul können ein containerartiges Behältnis ausgefüllt oder auch kleinere Einheiten errichtet werden. Mit diesen Grundmodulen entsteht ein variables System zur Container-Beschickung und -entnahme.

Das Containergefäß ist vorzugsweise mit öffenbaren, gas- und flüssigkeitsdichten Wandbauteilen versehen, wodurch eine Regeneration und Erneuerung der installierten Bauteile ermöglicht wird. Mit diesen Grundmodulen entsteht ein variables System zur Container-Beschickung und -entnahme.

Das Container Gefäß kann aus korrosionsfestem und $H_2$-dichtem verschweißbaren Kunststoffmaterial Verbundwerkstoffen (Folien) hergestellt werden.

In einer speziellen Ausführungsvariante der Erfindung kann für die Erhöhung der Effizienz des Systems der Druck im Systems durch die senkrechte Schichtung der Basisreaktoreinheiten je Containereinheit auf ca. 1,2 bar abs. erhöht werden.

Für die Reaktionseffizienz sind nicht nur die Druckerhöhung, die Feinperligkeit des einströmenden Gases, sondern auch die Verweilzeit in der Kolonne entscheidend. Hierdurch wird die Löslichkeit bzw. der Lösungsgrad der Gase in der wässrigen Suspension als Reaktionsraum beeinflusst.

Die Feinperligkeit wird durch den Einsatz von vorgemischtem Gas aus einer Vormischkammer sowie die anschließende Verteilung durch Membran-Diffusoren unterhalb des durchlässigen Kolonnenbodens erzielt. Gemäß einer bevorzugten Ausführungsform dienen dafür Membran-Tellerbelüfter gemäß Figur 8.

Durch den Einsatz von Gasleiteinsätzen (entspricht in der Form einer Förderschnecke d.h. kurze Segmente davon senkrecht in die Kolonne gestapelt siehe Anlage angepasste Zeichnung kommt) in den Kolonnen wird die Verweilzeit des aufsteigenden Gases bestimmt und verlängert. Die Löslichkeit der Gase wird hiermit zusätzlich zum erhöhten Druck verbessert.

Mit diesen Maßnahmen wird ein variabler Umsetzungsgrad der Ausgangsgase $CO_2$ und $H_2$ auch größer als 85% Methananteil erzielt.

Die Containerbehälter mit ihren inhaltlich platzierten Grundmodulen der Mehrfach-Reaktoren und der integrierten Mikro-Kolonnen des Hybrid-Reaktors können zu Verbünden in diverser Anzahl zu einem Makro-Reaktor (diverse Containerbehälter, wie oben beschrieben) zusammengeschaltet werden.

Durch gezielte Reihenschaltung und die Gasmengen- und qualitätsabhängige Umschaltung der Container-Behälter-Verbände wird ein Maximum des $CH_4$ Gehaltes im das System verlassenden Reaktionsendprodukt-Gas bis nahezu 99% igem Anteil erzielt.

Dadurch ist das System geeignet, wechselnden Gasmengen, wechselnden Gasqualitäten des Eingangsgemisch Gases auf der Beschickungsseite durch entsprechende Umschaltungsmaßnahmen anzupassen.

Das System ist durch diesen Aufbau mit variablen Schaltungsmöglichkeiten geeignet, bei Bedarf auch einen definierten $CH_4$-$CO_2$- Gehalt im Ausgangs-Gas herzustellen. Hiermit entsteht eine Möglichkeit, unterschiedliche Gaskonzentrationen der Reaktionsgase im Zustrom Bereich an verschiedene Anwendungsbereiche mit unterschiedlichen $CH_4$-Gehaltsanforderungen anzupassen.

Der Kern der Erfindung ist, den Reaktor mehrstufig und parallel in einem containerartigen Gehäuse zu realisieren. Das heißt es werden mehrere Körbe der beiden Komponenten übereinandergestellt und das in diversen parallel im Gehäuse aufgestellten Einheiten (also nicht nur die "Container" übereinander). Darüber hinaus wird die Gaszufuhr ebenfalls mehrstufig vorgenommen, zwischen den Körben, vorzugsweise nach jedem zweiten Einsatzkorb.

Die Erfindung soll anhand von Ausführungsbeispielen näher beschrieben werden, ohne sie auf diese Beispiele zu reduzieren.

**Ausführungsbeispiele**

In Figur 3 ist das Verfahren zur Anwendung des oben beschriebenen Generators Turbo-Methan beschrieben.

Hierbei handelt es sich um die Einkoppelung des Generators in den Gasweg der bestehenden Biogasanlagen auf dem Weg des Gases direkt zum BHKW bzw. über einen Zwischengasspeicher zum BHKW. Gasreinigung und Trocknung aus dem Bestand der Anlage bleiben auf diesem Wege vor dem BHKW integriert.

Das Biogas aus dem Fermenterprozeß wird hierbei entsprechend der Anforderung an den Methangehalt des BHKW(<= 75%) mit Wasserstoff aus einer beliebigen externen Quelle gemischt, so dass es dem Turbo Methan Generator zugeführt wird.

Herkömmliche BHKW in bestehenden Biogasanlagen können unter Berücksichtigung der erforderlichen Motormanagement Einstellungen bis zu einem Methan Gehalt von 75% im Brenngas arbeiten. Der Turbo Methan Generator stellt Brenngas mit einem Methangehalt von ca. 85% zur Verfügung. Um den Grenzwert von 75% Methan am BHKW nicht zu überschreiten wird das Brenngas aus Turbo Methan und das Fermentergas dementsprechend gemischt bzw. abgeglichen.

Mit der Bereitstellung von 75% Methan haltigem Gas am BHKW ist die Effizienzsteigerung an bestehenden Anlagen um ca. 50% möglich, wobei ca. 50% des üblichen $CO_2$ Gases aus dem Fermentergas in zusätzliches Methan umgewandelt wird. Durch die Koppelung mit weiteren Effizienz erhöhenden Maßnahmen s. Figur 3 kann für bestehende Biogasanlagen mit überschaubarem anlagentechnischen Aufwand durch Einsatz des Turbo Methan Generators in Koppelung mit einer sekundären Wasserstoffquelle eine Anlagen-Effizienz-Steigerung von mehr als 60% erzielt werden.

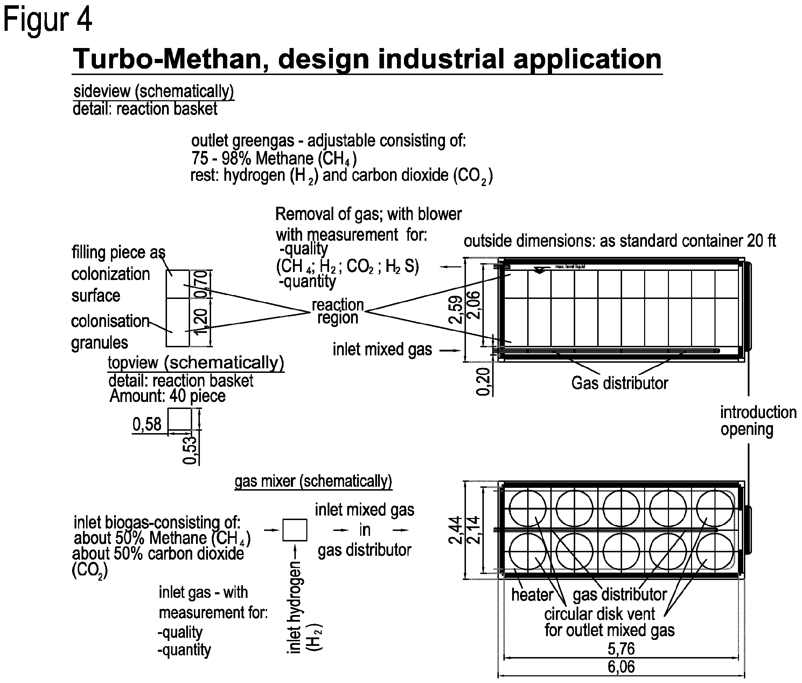

In der Figur 4 wird ein Horizontal- und ein Vertikal-Schnitt durch den als Container gestalteten Turbo-Methan-Reaktor dargestellt. Die so genannten reaction bascet genannten Mikrokolonnen sind hier schematisch dargestellt.

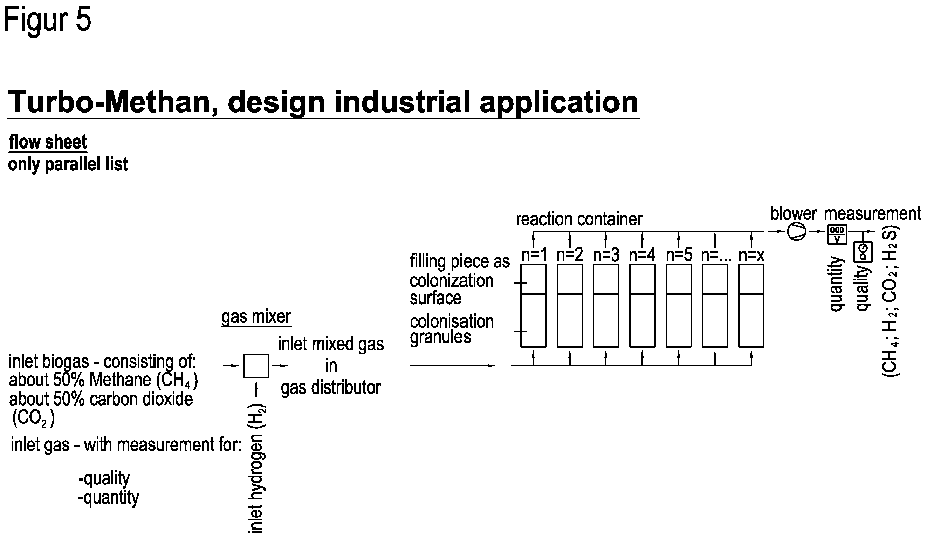

In der Figur 5 sind unterschiedliche Varianten zur Anpassungen des Grundbauteils der Mikrokolonnen oder auch gleichermaßen schaltbaren bspw. als 20 Fuß Container gefertigten Turbo Methan Reaktors an verschiedene Bedarfe der Gasmengen als auch der Gasqualität dargestellt.

Zur Anpassung an die Gasmengen werden die Mikrokolonnen oder die Container parallelgeschaltet.

Zur Anpassung an die Gasqualität, den Methangehalt werden die Mikrokolonnen bzw. die Container in Reihe geschaltet.

Zur Anpassung an beide Parameter, Gasmenge und Gasqualität, Methangehalt, wird eine kombinierte Parallel- und Reihenschaltung der Mikrokolonnen oder der Container vorgenommen.

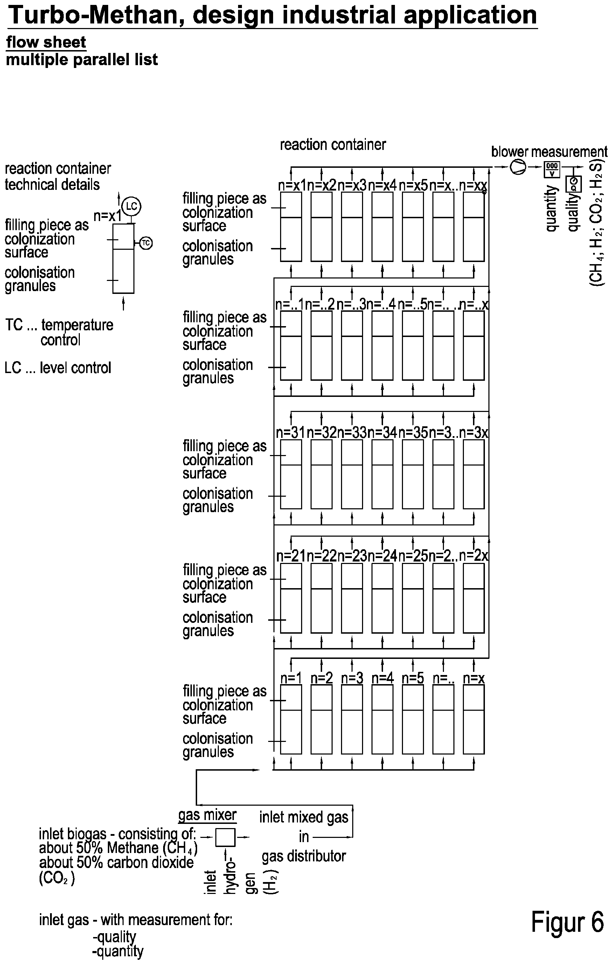

In der Figur 6 ist die maximale Anpassung durch Parallelschaltung an maximale Gasmengen dargestellt.

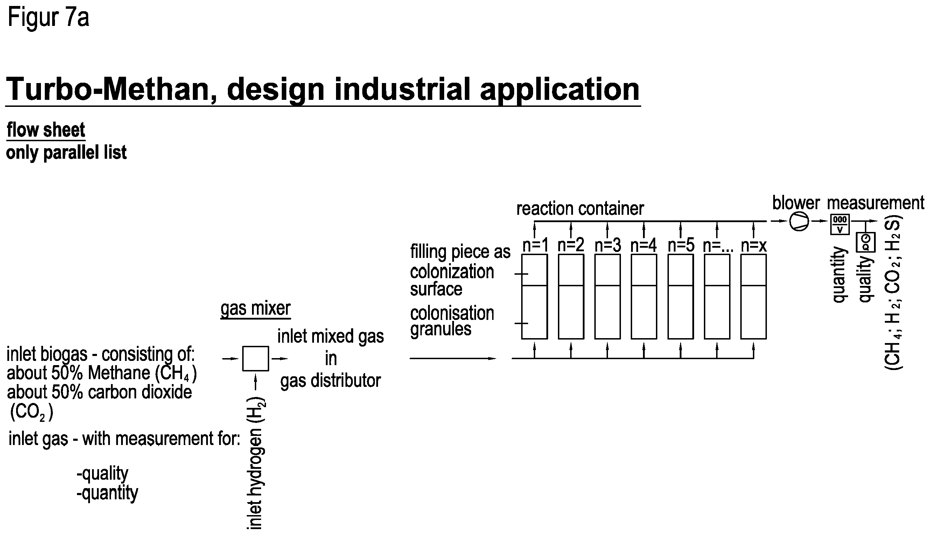

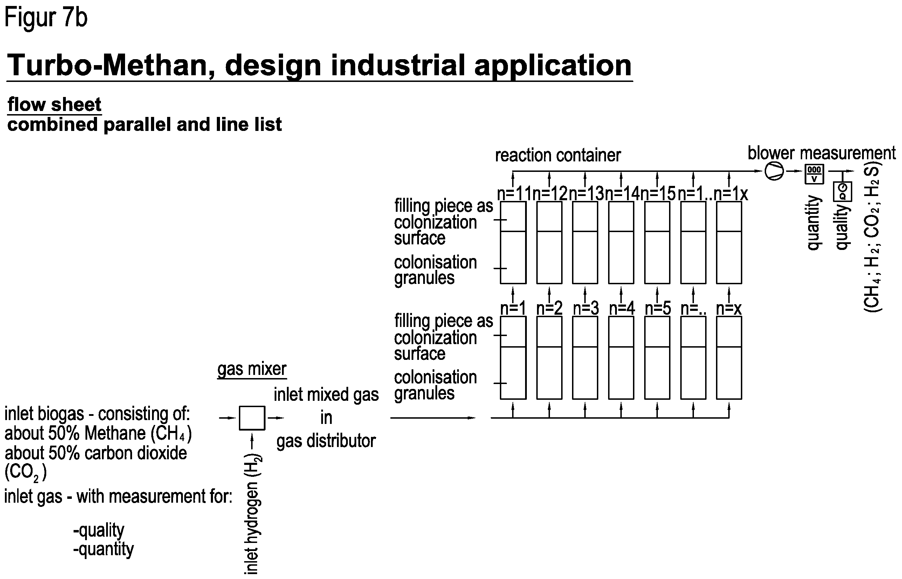

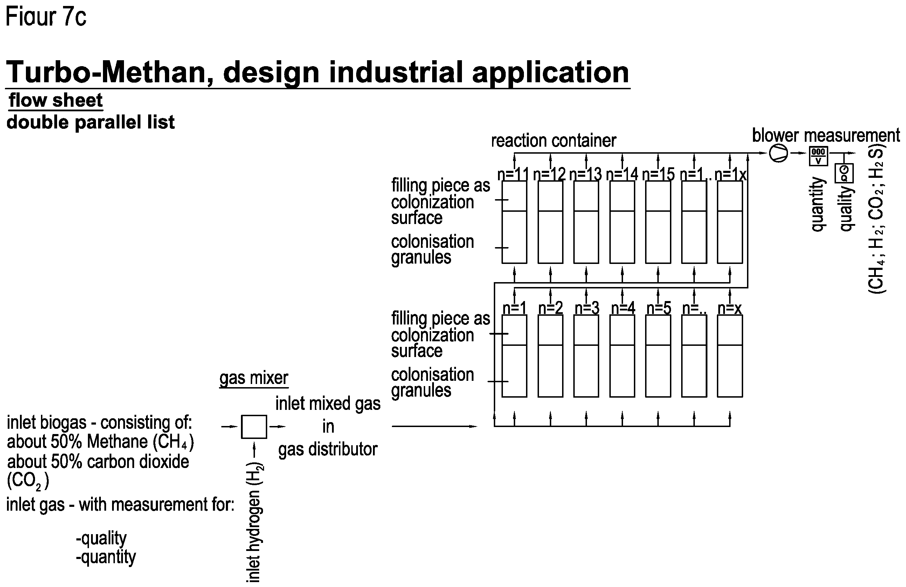

In der Figur 7 ist die Parallelschaltungsvariante aus Abbildung vergrößert dargestellt.

Die Bakterien, die zur Anwendung kommen können, sind - ohne den Anspruch auf Vollständigkeit zu erheben - in der folgenden Tabelle 1 aufgelistet.

**Tabelle 1**

| Am engsten verwandter Organismus | Ähnlichkeit (%) und NCBI Zugangsnummer |
|---|---|
| Methanosaeta concilii strain X16932 | %96 / KM408635.1 |
| Methanosaeta sp. | %96 / KP205578.1 |
| Unidentified archaeon clone | %93 / AJ831011.1 |
| Uncultured archaeon isolate | %92 / KC305601.1 |
| Uncultured Methanosarcinales archaeon clone | %93 / KF198727.1 |
| Uncultured archaeon clone | %97 / GU892615.1 |
| Methanofollis formosanus | %92 / NR042767.1 |
| Methanosphaerula palustris | %92 / NR074167.1 |
| Uncultured archaeon isolate | %98 / KC513450.1 |
| Uncultured Methanoculleus sp | %91 / GU583804.1 |
| Uncultured Methanofollis sp. | %93 / KP343676.1 |
| Uncultured Methanoculleus sp. | %93 / KC533587.1 |
| Uncultured archaeon clone | %96/ AB700446.1 |
| Uncultured Methanoculleus sp. | %96 / LC036210.1 |

(fortgesetzt)

| Am engsten verwandter Organismus | Ähnlichkeit (%) und NCBI Zugangsnummer |
|---|---|
| Uncultured archaeon isolate DGGE gel band | %98 / KC305610.1 |
| Methanobacterium petrolearium strain | %99 / NR113044.1 |
| Methanobacterium sp. enrichment culture clone | %98 / FJ984757.1 |
| Methanobacterium palustre strain | %99 / DQ649333.1 |
| Methanobacterium sp. enrichment culture clone | %99 / FJ984757.1 |
| Methanobacterium aarhusense strain | %94 / NR042895.1 |
| Uncultured Methanosaeta sp. | %98 / KJ522706.1 |
| Uncultured Methanosarcinales archaeon clone | %100 / EU586108.1 |
| Uncultured archaeon clone | %99 / KM213862.1 |
| Uncultured Methanolinea sp. | %99/ AB479405.1 |
| Uncultured Methanomicrobiales archaeon clone | %99 / JN394650.1 |
| Uncultured archaeon gene | %99 / LC108820.1 |
| Uncultured archaeon clone | %99 / KC736154.1 |
| Uncultured Methanomicrobiales archaeon clone | %95 / FN679176.1 |
| Uncultured Methanomicrobiales archaeon clone | %99 / FN679176.1 |
| Uncultured archaeon clone | %99 / LC108820.1 |

**Patentansprüche**

1. Vorrichtung zur Nutzung von Rest-$CO_2$ für die weitere $CH_4$-Produktion, umfassend Reaktoren, die anaerobe methanbildende Bakterien aus Schlamm Granulat und darüber Katalysatoren/mikrobielle Besiedlungsfläche aus organischen Kunststoffen, Mineralien oder metallischen Stoffen mit spezifisch sehr großer Oberfläche aufweisen, **dadurch gekennzeichnet, dass** die Reaktorräume mehrfach in einer Mikro-Kolonne zu einem Mehrfach-Reaktor aufgeschichtet sind.

2. Vorrichtung nach Anspruch 1, **dadurch gekennzeichnet, dass** die organischen Kunststoffe, Mineralien oder metallischen Stoffe eine spezifisch sehr große Oberfläche aufweisen.

3. Vorrichtung nach Anspruch 1 oder 2, **dadurch gekennzeichnet, dass** es sich bei den Katalysatoren um diverse die mikrobielle Besiedlungsoberfläche vergrößernde Schüttkörper aus Kunststoffen, Mineralien oder metallischen Stoffen handelt, die sich vorzugsweise in einem Stahlkorb geschichtet befinden.

4. Vorrichtung nach einem der Ansprüche 1 bis 3, **dadurch gekennzeichnet, dass** die Mikro-Kolonne jeweils eine Kombination mit der Schichtung Granulat-Katalysator/Mikrobieller-Siedlung-Reaktionsraum aufweist.

5. Vorrichtung nach einem der Ansprüche 1 bis 4, **dadurch gekennzeichnet, dass** die Schichtung senkrecht übereinander erfolgt, vorzugsweise in wechselbaren Einfüllschächten als Füllkörbe mit Flüssigkeits- und gasdurchlässigem Boden.

6. Vorrichtung nach einem der Ansprüche 1 bis 5, **dadurch gekennzeichnet, dass** die Mikro-Kolonnen als Einschub-System und mit Gleiteinrichtungen ein ausgestattetes Grundmodul darstellen, das sich zu einem Container Gefäß erweitern lässt.

7. Vorrichtung nach einem der Ansprüche 1 bis 6, **dadurch gekennzeichnet, dass** das Containergefäß öffenbaren, gas- und flüssigkeitsdichten Wandbauteilen versehen ist und / oder aus $H_2$-dichtem verschweißbaren Kunststoffmaterial Verbundwerkstoffen (Folien) hergestellt ist.

8. Vorrichtung nach einem der Ansprüche 1 bis 7, **dadurch gekennzeichnet, dass** die Mikro-Kolonnen Gasleiteinsätze aufweisen, die vorzugsweise mit der Schichtung als Spiral(Schnecken-)-Segmente schichtweise eingebracht sind.

9. Vorrichtung nach einem der Ansprüche 1 bis 8, **dadurch gekennzeichnet, dass** die Vorrichtung ein oder mehrere Gas-Vormischkammern aufweist, welche extern zentral platziert oder in den Container unterhalb der gasdurchlässigen Zwischenböden integriert sind.

10. Vorrichtung nach einem der Ansprüche 1 bis 9, **dadurch gekennzeichnet, dass** sich eine oder mehrere Membran-Diffusoren, vorzugsweise Membran-Tellerbelüfter, unterhalb des durchlässigen Kolonnenbodens befinden.

11. Verfahren zur Nutzung von Rest-$CO_2$ für die weitere $CH_4$-Produktion mittels der, **dadurch gekennzeichnet, dass** Biogas, welches $CH_4$ und $CO_2$ enthält durch die Vorrichtung gemäß einem der Ansprüche 1 bis 10 so geleitet wird, dass es zunächst die Schicht mit dem Schlamm-Granulat durchläuft und anschließend die Schicht mit den Katalysatoren/Besiedlungsflächen.

12. Verfahren nach Anspruch 11, **dadurch gekennzeichnet, dass** der Druck durch die senkrechte Schichtung der Basisreaktoreinheiten je Containereinheit auf ca. 1,2 bar erhöht wird.

13. Verwendung der Vorrichtung gemäß einem der Ansprüche 1 bis 10 zur Produktion eines Biogases, welches einen variablen Methangehalt entsprechend dem Verwendungszweck des finalen Gases am Ausgang der Vorrichtung realisiert, welcher zwischen dem Ursprungsgehalt und einem Methangehalt von ca. 98% liegen kann.

14. Verwendung der Vorrichtung gemäß den Ansprüchenl-10 zur Produktion eines Biogases mit einem für die Einspeisung in Gasnetze geeigneten Methangehalt von ca. 98%, wobei die erzeugte Gasmenge den bereitgestellten Wasserstoff Volumina angepasst wird

15. Verwendung von überschüssigem Strom zur Wasserstoffproduktion und der Nutzung des Wasserstoffs in der Vorrichtung gemäß der Vorrichtung nach einem der Ansprüche 1 bis 10 zur Erhöhung der Methankonzentration an bestehenden Biogasanlagen, Biomethananlagen und anaeroben Vergärungsanlagen.

**Figur 1**

Figur 2

Gas Flowmeter

Sampling Point

Steel Basket

Granular sludge

Glass bead layer

GAS FLOW CONTROLLERS

H₂   CO₂

EP 3 868 858 A1

## Figur 3

# Turbo-Methan

### Turbo-Methan
( Variante 1)

Biogasanlage

**Windkraft**

**Strom-Netz**

BHKW

Biogas
$CH_4$ 75%
$CO_2$ 24%

| Wave-Box | Kombi Max | Turbo-methan | Turbo Generator |
|---|---|---|---|

Stromnetzentlastung
bei Überschuß-Strom

Aufnahme
preiswerter
EE -Strom

über 60% Einsparungen

Biogas
$CH_4$ 52%
$CO_2$ 48%

$H_2$ 99%

$H_2$

Speicher

Elektrolyseur

Photovoltaik

EP 3 868 858 A1

# Figur 4

## Turbo-Methan, design industrial application

<u>sideview (schematically)</u>
<u>detail: reaction basket</u>

outlet greengas - adjustable consisting of:
75 - 98% Methane ($CH_4$)
rest: hydrogen ($H_2$) and carbon dioxide ($CO_2$)

filling piece as
colonization
surface

colonisation
granules

Removal of gas; with blower
with measurement for:
-quality
($CH_4$; $H_2$; $CO_2$; $H_2$ S)
-quantity

reaction
region

inlet mixed gas →

outside dimensions: as standard container 20 ft

max. level liquid

Gas distributor

<u>topview (schematically)</u>
detail: reaction basket
Amount: 40 piece

0,58

introduction
opening

<u>gas mixer (schematically)</u>

inlet biogas-consisting of:
about 50% Methane ($CH_4$)
about 50% carbon dioxide
($CO_2$)

inlet gas - with
measurement for:
-quality
-quantity

inlet hydrogen
($H_2$)

inlet mixed gas
→ in →
gas distributor

heater gas distributor
circular disk vent
for outlet mixed gas

5,76

6,06

EP 3 868 858 A1

# Figur 5

## Turbo-Methan, design industrial application

**flow sheet**
**only parallel list**

inlet biogas - consisting of:
about 50% Methane ($CH_4$)
about 50% carbon dioxide
($CO_2$)

inlet gas - with measurement for:

    -quality
    -quantity

inlet hydrogen ($H_2$)

<u>gas mixer</u>

inlet mixed gas
in
gas distributor

filling piece as
colonization
surface

colonisation
granules

reaction container

n=1  n=2  n=3  n=4  n=5  n=...  n=x

blower  measurement

quantity

quality

($CH_4$; $H_2$; $CO_2$; $H_2S$)

EP 3 868 858 A1

# Turbo-Methan, design industrial application

## flow sheet
## multiple parallel list

reaction container

blower measurement

quantity
quality
(CH$_4$; H$_2$; CO$_2$; H$_2$S)

reaction container
technical details

filling piece as
colonization
surface

colonisation
granules

n=x1

LC

TC

TC ... temperature
control

LC ... level control

filling piece as
colonization
surface

colonisation
granules

n=x1 n=x2 n=x3 n=x4 n=x5 n=x.. n=xx

filling piece as
colonization
surface

colonisation
granules

n=..1 n=..2 n=..3 n=..4 n=..5 n=.. n=..x

filling piece as
colonization
surface

colonisation
granules

n=31 n=32 n=33 n=34 n=35 n=3.. n=3x

filling piece as
colonization
surface

colonisation
granules

n=21 n=22 n=23 n=24 n=25 n=2.. n=2x

filling piece as
colonization
surface

colonisation
granules

n=1 n=2 n=3 n=4 n=5 n=.. n=x

gas mixer        inlet mixed gas

inlet biogas - consisting of:
about 50% Methane (CH$_4$)
about 50% carbon dioxide
(CO$_2$)

in

gas distributor

inlet
hydrogen
(H$_2$)

inlet gas - with measurement for:
        -quality
        -quantity

Figur 6

# Figur 7a

## Turbo-Methan, design industrial application

**flow sheet**
**only parallel list**

inlet biogas - consisting of:
about 50% Methane ($CH_4$)
about 50% carbon dioxide
($CO_2$)

inlet gas - with measurement for:

    -quality
    -quantity

inlet hydrogen ($H_2$)

**gas mixer**

inlet mixed gas
in
gas distributor

filling piece as
colonization
surface

colonisation
granules

reaction container

n=1 n=2 n=3 n=4 n=5 n=... n=x

blower measurement

quantity
quality

($CH_4$; $H_2$; $CO_2$; $H_2S$)

EP 3 868 858 A1

# Figur 7b

# Turbo-Methan, design industrial application

## flow sheet
## combined parallel and line list

reaction container     blower measurement

n=11 n=12 n=13 n=14 n=15 n=1..n=1x

filling piece as colonization surface

colonisation granules

quantity

quality

($CH_4$; $H_2$; $CO_2$; $H_2S$)

n=1 n=2 n=3 n=4 n=5 n=.. n=x

filling piece as colonization surface

colonisation granules

gas mixer

inlet mixed gas in gas distributor

inlet biogas - consisting of:
about 50% Methane ($CH_4$)
about 50% carbon dioxide ($CO_2$)

inlet gas - with measurement for:

   -quality
   -quantity

inlet hydrogen ($H_2$)

Fiour 7c

# Turbo-Methan, design industrial application
## flow sheet
## double parallel list

reaction container

blower measurement

n=11 n=12 n=13 n=14 n=15 n=1.. n=1x

filling piece as colonization surface

colonisation granules

quantity

quality

$(CH_4; H_2; CO_2; H_2S)$

n=1 n=2 n=3 n=4 n=5 n=.. n=x

filling piece as colonization surface

colonisation granules

gas mixer

inlet mixed gas in

gas distributor

inlet biogas - consisting of:
about 50% Methane $(CH_4)$
about 50% carbon dioxide $(CO_2)$

inlet gas - with measurement for:

-quality
-quantity

inlet hydrogen $(H_2)$

EP 3 868 858 A1

**Europäisches Patentamt**
**European Patent Office**
**Office européen des brevets**

# EUROPÄISCHER RECHERCHENBERICHT

Nummer der Anmeldung

EP 20 15 8085

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2013 001689 A1 (REULE WALDEMAR E [DE]) 31. Juli 2014 (2014-07-31) * Absätze [0005] - [0008], [0019], [0024], [0026], [0027], [0033] - [0038], [0040] * * Abbildungen 1,3 * ----- | 1-4,7, 11,13-15 | INV. C12M1/107 C12M1/12 C12M1/00 C12M1/34 |
| A | DE 10 2010 043630 A1 (GICON GROSMANN INGENIEUR CONSULT GMBH [DE]) 26. Mai 2011 (2011-05-26) * Absätze [0020] - [0033]; Abbildungen 1,3,4,6 * ----- | 1,2,11, 13-15 | |
| A | DE 10 2008 037402 A1 (SCHMACK BIOGAS AG [DE]) 1. April 2010 (2010-04-01) * Abbildungen 11-26 * ----- | 1-15 | |

RECHERCHIERTE SACHGEBIETE (IPC)

C12M

Der vorliegende Recherchenbericht wurde für alle Patentansprüche erstellt

| Recherchenort | Abschlußdatum der Recherche | Prüfer |
|---|---|---|
| Berlin | 28. Juli 2020 | Böhm, Ingo |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 20 15 8085

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-07-2020

| Im Recherchenbericht angeführtes Patentdokument | Datum der Veröffentlichung | Mitglied(er) der Patentfamilie | Datum der Veröffentlichung |
|---|---|---|---|
| DE 102013001689 A1 | 31-07-2014 | KEINE | |
| DE 102010043630 A1 | 26-05-2011 | KEINE | |
| DE 102008037402 A1 | 01-04-2010 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **SABATIER.** *Senderens Compte Rendu Acad. Sci.,* 1902, vol. 134, 689
- **MATTHIAS BRAKE.** Erdgasleitungen als Speicher für Windenergie. *Telepolis,* 18. April 2011
- **COSTA K ; LEIGH A.** Metabilic versatility in methanogens, current option. *Biotechnology,* 2014, vol. 29, 70-75